Europäisches Patentamt

(19) European Patent Office

Office européen des brevets

(11) Publication number: **0 096 934**
**B1**

(12) **EUROPEAN PATENT SPECIFICATION**

(45) Date of publication of patent specification: 26.11.86

(51) Int. Cl.⁴: **A 61 K 49/02**

(21) Application number: 83200830.4

(22) Date of filing: 07.06.83

(54) Radiographic imaging agents.

(30) Priority: 10.06.82 US 387137

(43) Date of publication of application:
28.12.83 Bulletin 83/52

(45) Publication of the grant of the patent:
26.11.86 Bulletin 86/48

(84) Designated Contracting States:
AT BE CH DE FR GB IT LI LU NL SE

(56) References cited:
GB-A-1 489 330
GB-A-1 541 070

JOURNAL OF NUCLEAR MEDICINE, vol. 21, no.
4, April 1980, pages 366-370 A.J. TOFE et al.:
"Gentisic acid: a new stabilizer for low tin
skeletal imaging agents: concise
communication"

CHEMICAL ABSTRACTS, vol. 92, no. 18, 5th
May 1980, page 406, no. 153120p, Columbus,
Ohio, US A.J. TOFE et al.: "Antioxidant
stabilization of bone agents"

(73) Proprietor: MALLINCKRODT, INC.(a Missouri
corporation)
675 McDonnell Boulevard P.O. Box 5840
St. Louis Missouri 63134 (US)

(72) Inventor: Vanduzee, Barry Foster
1191 Highcliff Court
Cincinnati Ohio 45224 (US)

(74) Representative: Eyles, Christopher Thomas et al
BATCHELLOR, KIRK & EYLES 2 Pear Tree Court
Farringdon Road
London, EC1R 0DS (GB)

Courier Press, Leamington Spa, England.

**Description**

This invention relates to radiodiagnostic agents, including compositions which can be reconstituted to form radiodiagnostic agents particularly useful for skeletal imaging (bone scanning).

Scintigraphic skeletal imaging and similar radiographic techniques for visualizing other tissues are finding ever-increasing application in biological and medical research and in diagnostic procedures. Generally, scintigraphic procedures involve the preparation of radioactive agents which, upon introduction into a biological subject, become localized in specific organs, tissues, or skeletal structures that are under study. When so localized, traces, plots, or scintiphotos of the distribution of the radiographic materials can be made by various radiation detectors, e.g., traversing scanners and scintilation cameras. The distribution and corresponding relative intensity of the detected radioactive material not only indicates the position occupied by the tissue in which the radionuclide is localized, but also indicates the presence of aberrations, pathological conditions, and the like.

In general, depending on the type of radionuclide used and the organ of interest, a scintigraphic imaging agent as used in a hospital comprises a radionuclide, a carrier agent designed to target the specific organ, various auxiliary agents which affix the radionuclide to the carrier, water or other delivery vehicles suitable for injection into, or aspiration by the patient, physiologic buffers and salts, and the like. The carrier attaches or complexes with the radionuclide and localizes the material in the location where the carrier naturally concentrates in a biologic subject.

Technetium-99m ($^{99m}$Tc) is a radionuclide which is widely used in tissue imaging agents. This radionuclide is conveniently available commercially in the oxidized pertechnetate form ($^{99m}$TcO$_4$$^-$, hereinafter "pertechnetate-Tc99m"). However, the technetium in pertechnetate has a valence state of +7 and, thus, will not complex with the most commonly used carriers for radionuclide tissue imaging. This problem is easily overcome by reducing the technetium to what is believed to be the +3, +4, and/or +5 valence state. Thus, technetium-labeled imaging agents are generally prepared by admixing pertechnetate-Tc99m isotonic saline solution with a technetium reductant (reducing agent) such as the stannous, ferrous, or chromous salt of sulfuric or hydrochloric acid, and the desired carrier agent for targeting the organ of interest. For example, organophosphonates are known as suitable carrier agents which target technetium radionuclide to bone tissue. U.S. Patent 3,983,227, Tofe and Francis, issued September 28, 1976, discloses the use of reducing salts with radioactive pertechnetate-Tc99m solutions and organophosphonate bone-seeking carriers to prepare skeletal imaging agents.

Technetium-containing scintigraphic imaging agents are known to be unstable in the presence of oxygen, primarily since oxidation of the reductant and/or the technetium destroys the reduced technetium/targeting carrier complex. Accordingly, such imaging agents are generally made oxygen-free by saturating the compositions with oxygen-free nitrogen gas or by preparing the agents in an oxygen-free atmosphere. Stabilization of imaging agents can also be achieved through chemical means. German Offenlegungsschrift 2,618,337, Tofe, published November 11, 1976, discloses the use of ascorbate stabilizers with technetium imaging agents. U.S. Patent 4,232,000, Fawzi, issued November 4, 1980, discloses the use of gentisyl alcohol as a stabilizer for technetium imaging agents. Similarly. U.S. Patent 4,233,284, Fawzi, issued November 11, 1980, discloses the use of gentisic acid as a stabilizer, as does an article by Tofe *et al*, Journal of Nuclear Medicine, *21* (4), p. 366—370 (1980).

Commercial products for use in skeletal imaging are generally provided in liquid or dry powder mixture imaging "kits" with vials containing phosphate or phosphonate bone seeking carriers. Such kits typically contain relatively substantial amounts of a reducing metal salt, such as stannous chloride. For example, one such kit, Osteoscan-HDP®, comprises about 3 mg of the sodium salts of methanehydroxydiphosphonic acid (HMDP), about 0.24 mg stannous chloride, and about 0.84 mg gentisic acid stabilizer. The advantages of using high stannous concentrations are discussed in the aforementioned article by Tofe et al, Journal of Nuclear Medicine, *21* (4), p 366—370 (1980).

It has now been discovered that tissue imaging kits containing lower amounts of a stannous reducing agent than have heretofore been employed yield imaging agents with improved performance. Such kits have a high ratio of moles diphosphonate to moles stannous salt.

This invention relates to a skeletal imaging kit, comprising:

(1) a diphosphonate bone-seeking carrier selected from methanediphosphonic acid, methanehydroxydiphosphonic acid, and the pharmaceutically-acceptable salts and mixtures thereof; and
(2) an effective amount of a stannous reductant; wherein the molar ratio of the diphosphonate to stannous tin is at least 65:1.

This invention is based on the discovery that a diphosphonate-containing skeletal imaging kit containing low levels of stannous reductant yields a skeletal imaging agent having improved performance as compared with compositions known in the art. This improvement is manifested through faster blood clearance and higher skeletal uptake of the technetium imaging agent, i.e. the bone-targeting carrier, complexed with technetium, is more quickly and more highly concentrated (distributed) in bone tissue as compared to surrounding soft tissues in the body.

As used herein, the term "imaging" refers to all radiographic tissue imaging processes for which the instant compositions may be used, including (but not limited to) skeletal imaging. The term "imaging agent" herein refers to compositions useful for imaging, including (but not limited to) skeletal imaging, such compositions comprising the product of admixing pertechnetate-Tc99m, or other useful radioisotope, to an imaging kit comprising a tissue-seeking carrier, stannous reductant, and, optionally, a stabilizer. Hence, the term "imaging kit" or "kit", refers to the imaging agent before addition of a solution of pertechnetate-Tc99m, or similar radionuclide.

The components of the composition herein, as well as methods of producing the composition, are described below. The quantity of these components incorporated into a preferred kit is enough to form multiple doses of imaging agent, as when reconstituted with a pertechnetate solution containing about 3.7 $10^7$ to $1.4810^{10}$ Bq (1 to 400 mCi) of technetium-Tc99m. (The number of doses ultimately obtained from such a kit depends upon such factors as the weight of the dosed subject and the type of tissue to be imaged.) Generally, then, a preferred kit comprises:

(a) an amount of diphosphonate carrier sufficient to target the technetium in a pertechnetate solution containing from about 3.7 $10^7$ to $1.4810^{10}$ Bq (about 1 to 400 mCi) of technetium-Tc99m;

(b) an effective amount of stannous reductant sufficient to reduce the technetium in a pertechnetate solution containing from about 3.7 $10^7$ to $1.4810^{10}$ Bq (about 1 to 400 mCi) technetium-99m, and

(c) an amount of stabilizer sufficient to prevent oxidation of the reductant and the reduced technetium-99m.

Components

Diphosphonate bone-seeking carriers useful in the instant invention include methanediphosphonic acid (MDP), methanehydroxydiphosphonic acid (HMDP), and pharmaceutically-acceptable salts and mixtures thereof. These compounds are disclosed in U.S. Patent 3,983,227, Tofe et al., issued September 28, 1976. U.S. Patent 4,247,534, Bevan, issued January 27, 1981, also discloses oxidronate disodium, the disodium salt of HMDP, as a skeletal imaging carrier and for use in agents that image myocardial infarcts. HMDP and its pharmaceutically-acceptable salts are preferred carriers for use herein.

In order for these targeting carriers to be useful with technetium, commercially available technetium (pertechnetate) must be reduced to form trivalent, tetravalent, and/or pentavalent technetium, which is then available to attach or complex with the targeting carrier. Reducing metal cations, such as stannous ion ($Sn^{+2}$) are known reductants for reducing the technetium in imaging compositions. The present invention incorporates one or more water-soluble, pharmaceutically-acceptable compounds which provide stannous ion when in solution, e.g., stannous chloride, stannous fluoride, stannous sulfate, herein referred to as "reductant" or "stannous reductant." Stannous chloride ($SnCl_2$) is particularly preferred.

A sufficient amount of reductant must be included in an imaging kit to ensure complete reduction of the technetium-99m added in forming the imaging agent. This amount, herein "effective amount", is greater than or equal to (not less than) the stoichiometric amount to reduce all of the technetium in the pertechnetate to be added to the imaging kit, i.e., when the reductant is dissolved, there must be enough stannous ion in solution to reduce technetium (+7) to a lower valence state, facilitating complexation with a diphosphonate carrier. Preferably, the effective amount is at least two times the molar amount of technetium to be added to the kit. The specific quantity of stannous reductant incorporated into an imaging kit encompassed by this invention may vary according to such factors as the molecular weight of the salt, the amount of pertechnetate to be added to the kit, the desired storage time of the agent made from the kit, the presence of oxidants in the agent, and the presence of optional anti-oxidant stabilizers, as discussed below. In the present invention, the effective amount of reductant is such that the ratio of moles of diphosphonate to moles of stannous tin contained in the kit is not less than about 65:1. Preferably the effective amount is such that the ratio of moles is not less than about 75:1. The molar ratio should not exceed about 2,000:1. As used herein, "stannous tin" refers to elemental $Sn^{+2}$ contained in the reductant compound.

Although optional, the imaging kits of this invention preferably contain a stabilizing amount of a stabilizer material to prevent or inhibit the oxidation of the reductant (e.g., oxidation of $Sn^{+2}$ to $Sn^{+4}$) during storage and/or to inhibit or reduce the reoxidation of reduced technetium-99m and/or to reduce the formation of technetium-labeled impurities which may form during use of the compositions. The presence of a stabilizer is especially preferred when the low levels of reductant encompassed by this invention are used in multi-dose kits. The stabilizers optionally used herein are characterized by their toxicological acceptability under the conditions of use, their ability to stabilize the product for a reasonable period of storage and/or under usage conditions, and by their substantial non-interference with the delivery of the technetium radionuclide to bone mineral.

Stabilizers that meet the foregoing requirements and that are quite suitable for intravenous injection include hydroquinone, gentisyl alcohol, gentisic acid, ascorbic acid, erythorbic acid, and their water-soluble salts and esters. Gentisic acid, ascorbic acid, erythorbic acid, and their sodium salts are all known, commercially-available materials. The following documents describe useful stabilizers: U.S. Patent 4,232,200, Fawzi, issued November 4, 1980 (gentisyl alcohol); U.S. Patent 4,233,284, Fawzi, issued November 11, 1980 (gentisic acid); U.S. Patent 4,229,427, Whitehouse, issued October 21, 1980 (hydroquinone); and German Offenlegungsschrift 2,618,337, Tofe, published November 11, 1976 (ascorbic acid).

The sodium salt of ascorbic acid is a preferred stabilizer for use in a multi-dose dry-powder embodiment of this invention. Also preferred are the "reductate" stabilizers described in concurrently filed EP—A—96930 "Stable Radiographic Imaging Agents," Fawzi, et al.

As is known in the literature, stabilizer materials such as ascorbic acid can chelate/complex with technetium and cause it to be deposited in uncalcified soft tissue. Since the user of a kit encompassed by the present invention will wish to avoid all unnecessary deposition of technetium in soft tissue, it will be appreciated that the amount of stabilizer material optionally included should not be so great as to overshadow the bone-directing effect of the diphosphonate carrier thereby interfering with the bone scan. Appropriate, non-interfering amounts of stabilizer materials for use in combination with the diphosphonates may vary according to the diphosphonate and/or stabilizer used. Guidelines for determining such amounts are known in the art.

Composition and methods

The composition of this invention comprises:

(1) a diphosphonate carrier;
(2) a stannous reductant, and optionally
(3) a stabilizer;

wherein the molar ratio of diphosphonate to stannous tin is greater than or equal to about 65:1, preferably greater than or equal to about 75:1. This "molar ratio," or "[DP/Sn]" herein, is the ratio of the number of moles of diphosphonate present in the composition to the number of moles of stannous tin present.

A single unit-dose kit (that may be reconstituted to form an amount of imaging agent suitable for a single injection) for diphosphonates useful in the invention, containing no more than the maximum quantity of reductant encompassed by this invention, comprises at least from about $2\times10^{-7}$ to about $2\times10^{-6}$ moles of diphosphonate and about $4\times10^{-9}$ moles stannous tin.

The imaging agents made with the kits of this invention are intended for intravenous injection into humans or lower animals. Accordingly, appropriate manufacturing and operating conditions are employed so as to provide suitably sterile, pyrogen-free compositions. Although not necessary to the practice of the present invention, it is preferable to use a pharmaceutically-acceptable extender or filler to dilute the reducing and diphosphonate salts in order to simplify metering the requisite small quantities of such salts. Sodium chloride and glucose are preferred; sodium chloride is especially preferred inasmuch as its addition will assure that the resulting agent is at least isotonic even if the pertechnetate-Tc99m solution is hypotonic (as is the case when it must be diluted with sterile water to reduce its activity).

The compositions of the present invention can be prepared by simply dry mixing the technetium reductant and the diphosphonate carrier. The optional stabilizer can also be dry-blended into such mixtures, as can any additional, non-interfering agents such as sodium chloride. Such compositions are preferably placed in sterile vials fitted with a rubber septum, thereby facilitating mixing with a pertechnetate-Tc99m solution and convenient use in the hospital. The vials are preferably nitrogen-filed as an added protection against oxidation of the technetium reducing metal salt on storage.

In another mode, the compositions herein can be provided as aqueous solutions in sterile, pyrogen-free water. Preferably, the water is deoxygenated and the composition is stored under nitrogen, thereby minimizing undesirable oxidation of the pertechnetate reductant on storage. Since the reductant is more prone to oxidize in solution than in the dry powder and freeze-dried composition forms, it is preferred that aqueous compositions contain a stabilizer.

In a preferred mode, the compositions herein can be provided in freeze-dried (lyophilized) form. Such compositions are prepared by co-dissolving the diphosphonate carrier and the technetium reductant in an aqueous solution, together with any desired optional stabilizers, and freeze-drying the composition using standard equipment. Preferably, sterile, deoxygenated water is used in processing and the product is stored under nitrogen. Although somewhat more complicated to manufacture than the dry mixture product, the freeze-dried product offers the advantage that water-insoluble particulate matter which might be present in the raw materials can be removed by filtration prior to the freeze drying step.

A preferred method of lyophilizing involves dissolving the carrier and stabilizer first, and then dissolving the reductant. A preferred kit is produced by incorporating an ascorbate or reductate stabilizer and adjusting the carrier/reductant/stabilizer solution to about pH 6.0 through the addition of sodium hydroxide. If no stabilizer is used, or if a stabilizer is used comprising hydroquinone, gentistic acid, gentisyl alcohol, and/or their pharmaceutically-acceptable salts and esters, the carrier/reductant solution is preferably adjusted to about pH 4.5 through the addition of sodium hydroxide. These processes are described in the following European Patent Applications, concurrently filed therewith: Application No. 83200829.6 (Publication No. 0096933) "Process for Making a Lyophilized Product For Use in Skeletal Imaging". Van Duzee and Degenhardt (process with ascorbate/reductate stabilizer); Application No. 83200828.8 (Publication No. 0096932) and "Process for Making a Lyophilized Product For Use in Skeletal Imaging" Van Duzee (process with optional gentisate stabilizer).

The compositions of this invention are dissolved with a pertechnetate-Tc99m isotonic solution from a commercial technetium source to yield an imaging agent suitable for intravenous injection. The stability of such imaging agents is ample under ordinary hospital conditions. Administration is preferably done within about eight hours after addition of the pertechnetate-Tc99m solution. Preferably, the concentration of reagents and technetium radionuclide is sufficient that about 1 milliliter of the solution is used in an adult of about 50—100 kg body weight. One milliliter of solution is preferably injected intravenously over a period of about 30 seconds. The total dosage of radionuclide for a sharp skeletal or myocardial infarct scan ranges from $18.5 \cdot 10^7$ to $111.10^7$ Bq 5 mCi to 30 mCi), preferably from $37.10^7$ to $74.10^7$ Bq (10 mCi to 20 mCi). See also U.S. Patent 4,234,562, Tofe et al, issued November 18, 1980; and U.S. Patent 4,247,534 Bevan issued January 27, 1981.

The following non-limiting examples illustrate the composition, product, and use of the present invention.

Example 1

An imaging kit, encompassed by the present invention, was produced with the following ingredients:

| Component | Quantity in bulk | Quantity in kit |
|---|---|---|
| disodium salt of HMDP | 300. mg | 3.0 mg |
| stannous chloride | 3.2 mg | 0.032 mg |
| gentisic acid | 84. mg | 0.84 mg |
| sodium chloride | 3000. mg | 30.0 mg |

The HMDP, gentisic acid, and sodium chloride ere dissolved in sterile, nitrogen-purged (deoxygenated) water. After dissolution of those components, the stannous chloride was dissolved in the solution. Sodium hydroxide was added to adjust the pH to 4.5. Sterile, deoxygenated water was added to bring the solution volume to 100 ml.

One milliliter aliquots of the solution were placed in sterile, nitrogen purged vials. The vials were then freeze-dried (lyophilized) in a commercial lyophilizer, stoppered and sealed. The kit composition had a [DP/Sn] of about 75.

A scanning agent is prepared using this kit by adding about 5 ml of a pertechnetate-Tc99m physiological saline solution, with an activity of $2.775.10^5$ Bq (about 75 mCi), from a commercial technetium source. The vial is agitated until the kit components are dissolved. About 1 ml of the agent is slowly injected, over a period of about 30 seconds, into an adult human subject weighing about 75 kg. Excellent skeletal images are then obtained using a scintillation camera.

In the kit prepared above, gentisyl alcohol, hydroquinone, ascorbic acid, erythorbic acid, 6-bromo-6-deoxyascorbic acid, 6-chloro-6-deoxyascorbic acid, reductic acid, 5-methyl-reductic acid, and the sodium salts thereof are, respectively, used instead of gentisic acid, with substantially similar results. However, when an ascorbate or reductate stabilizer is used, the pH of the carrier/reductant solution should be adjusted to about 6.0 rather than 4.5.

Example II

An imaging kit encompassed by the present invention is produced with the following ingredients:

| Component | Quantity in bulk | Quantity in kit |
|---|---|---|
| disodium salt of MDP | 200.0 mg | 2.0 mg |
| stannous chloride | 2.6 mg | 0.026 mg |
| sodium chloride | 600 mg | 6.0 mg |

Kits are made according to the process detailed in Example I, with a [DP/Sn] of about 65. Excellent skeletal images are obtained when this kit is used to prepare a scanning agent, and the agent is injected, as in Example I.

In the foregoing kit, stannous fluoride, stannous sulfate, stannous citrate, and stannous tartrate are, respectively, used instead of stannous chloride, in an amount sufficient to yield a [DP/Sn] of about 65, with substantially similar results. Aslo, in the foregoing kit, the amount of reductant used in varied so that the kit has a [DP/Sn] of greater than 65, with substantially similar or better results.

Lyophilized kits were made, according to the process of Example I, with the following compositions:

TABLE I

| Kit | Carrier (disodium salt thereof) | Quantity of carrier per vial | Quantity of $SnCl_2$ per vial | [DP/Sn] |
|---|---|---|---|---|
| A | ethane-1-hydroxy-1, 1-diphosphonic acid (EHDP) | 5.9 mg | .02 mg | 220 |
| B | EHDP | 5.9 mg | .04 mg | 110 |
| C | EHDP | 5.9 mg | .08 mg | 56 |
| D | EHDP | 5.9 mg | .12 mg | 37 |
| E | EHDP | 5.9 mg | .16 mg | 28 |
| F | HMDP | 3.0 mg | .016 mg | 150 |
| G | HMDP | 3.0 mg | .032 mg | 75 |
| H | HMDP | 3.0 mg | .080 mg | 30 |
| I | HMDP | 3.0 mg | .112 mg | 22 |
| J | HMDP | 3.0 mg | .240 mg | 10 |
| K | MDP | 10.0 mg | .05 mg | 170 |
| L | MDP | 10.0 mg | .10 mg | 86 |
| M | MDP | 10.0 mg | .40 mg | 22 |
| N | MDP | 10.0 mg | .85 mg | 10 |

Each kit also included about 0.84 mg gentisic acid as a stabilizer.

(Unlike Example I, kits K through N containing MDP were prepared in a process where the pH of the diphosphonate/$SnCl_2$ solution was adjusted to about 7.0, rather than 4.5.)

Each kit vial was reconstituted by addition of 5 ml of a pertechnetate-Tc99m physiological saline solution with an average activity of approximately 85.1 $10^7$ Bq (23 mCi). A 50 microliter dose of each imaging agent thus formed was injected into a fasted rat of an average weight of about 200 g. One dose of an imaging agent was injected into each rat to be studied, with four rats injected for each of kits A—I, K and L, and three rats for each of kits M and N. Several kits of composition J (equivalent to Osteoscan-HDP®) were evaluated in a series of experiments similar to the experiment described herein.

Scintiscans were taken of rats that were injected with agents made from each kit described in Table II, above. The rats were then sacrificed and blood, muscle, and bone tissue removed and placed in tared scintillation counting vials. The samples were weighed and radioassayed, along with a control sample of the original imaging agent made from each kit, using a gamma-scintillation spectrometer.

Table II, below, summarizes the distribution of the technetium-99m imaging agent in each rat's body, as a function of the [DP/Sn] of the imaging kit used to produce the agent. This distribution is a function of the technetium-99m retention by various body tissues and is recorded in Table II as the relative bone to muscle retention ratio and the relative bone to blood retention ratio.

6

TABLE II

| Kit | Carrier | [DP/Sn] | Bone/Muscle | Bone/Blood |
|---|---|---|---|---|
| A | EHDP | 220 | 295 | 42 |
| B | EHDP | 110 | 259 | 37 |
| C | EHDP | 56 | 231 | 34 |
| D | EHDP | 37 | 247 | 35 |
| E | EHDP | 28 | 232 | 35 |
| F | HMDP | 150 | 1116 | 185 |
| G | HMDP | 75 | 959 | 199 |
| H | HMDP | 30 | 761 | 134 |
| I | HMDP | 22 | 723 | 123 |
| J | HMDP | 10 | 610 | 85 |
| K | MDP | 170 | 515 | 88 |
| L | MDP | 86 | 417 | 75 |
| M | MDP | 22 | 224 | 41 |
| N | MDP | 10 | 208 | 33 |

(Data for kit J represents the average of several experiments using a composition equivalent to Osteoscan-HDP®.)

The data from kits A through E, containing ethane-1-hydroxy-1, 1-diphosphonic acid (EHDP), does not demonstrate any definite relationship between distribution of the imaging agent and the [DP/Sn] of the corresponding kit. Thus, skeletal imaging is not demonstrably improved by increasing the [DP/Sn] of imaging kits containing EHDP.

However, the data for kits F—J (HMDP) and kits K—N (MDP) clearly demonstrates the effect on distribution of a technetium-99m imaging agent as a function of the [DP/Sn] of the kit used to produce the agent. In particular, at the higher values of [DP/Sn], the imaging agent is concentrated more heavily in bone tissue as compared to blood and other soft tissue. Thus, imaging agents comprising certain diphosphonate carriers and stannous reductant, with a [DP/Sn] of at least about 65, yield imaging agents with improved imaging qualities, i.e., increased bone/soft tissue retention and enhanced blood clearance.

**Claims**

1. An imaging kit, characterized in that it comprises:

(a) a carrier selected from methanediphosphonic acid, methanehydroxydiphosphonic acid, and the pharmaceutically-acceptable salts and mixtures thereof; and

(b) an effective amount of stannous reductant; wherein the molar ratio of said carrier to said stannous tin is at least 65:1.

2. An imaging kit, according to Claim 1, characterized in that said molar ratio is at least 75:1.

3. An imaging kit, according to Claim 1 or 2, characterized in that said stannous reductant is stannous chloride.

4. An imaging kit, according to any of Claims 1—3, characterized in that it further comprises a stabilizing amount of a stabilizer selected from gentisic acid, gentisyl alcohol, and the pharmaceutically-acceptable salts thereof; ascorbic acid, erythorbic acid, 6-bromo-6-deoxyascorbic acid, 6-chloro-6-deoxy-ascorbic acid, reductic acid, 5-methyl reductic acid, and the nicotinic acid and nicotinamide complexes thereof and the pharmaceutically-acceptable salts thereof; and mixtures thereof.

5. An imaging kit, according to any of Claims 1—4, characterized in that

(a) said carrier is selected from methanehydroxydiphosphonic acid and the pharmaceutically-acceptable salts and mixtures thereof; and

(b) said kit includes a stabilizer selected from gentisic acid, ascorbic acid, 6-bromo-6-deoxyascorbic acid, pharmaceutically-acceptable salts and mixtures thereof.

7

6. An imaging kit, according to any of Claims 1—5, characterized in that it comprises:
(a) 3 mg of the sodium salts of methanehydroxydiphosphonic acid;
(b) 0.02 mg of stannous chloride; and
(c) from .25 mg to 1.0 mg of ascorbic acid or the sodium salt thereof.
7. An imaging kit, according to any of Claims 1—5, characterized in that it comprises:
(a) 10 mg of the sodium salts of methanediphosphonic acid;
(b) 0.02 mg of stannous chloride; and
(c) from .25 mg to 1.0 mg of ascorbic acid or the sodium salt thereof.

**Patentansprüche**

1. Sichtbarmachungsausrüstung, dadurch gekennzeichnet, daß sie
(a) einen unter Methandiphosphonsäure, Methanhydroxydiphosphonsäure und deren pharmazeutisch geeigneten Salzen und Gemischen ausgewählten Träger und
(b) eine wirksame Menge Zinn(II)-Reduktionsmittel aufweist, wobei das Molverhältnis des Trägers zu dem Zinn(II) wenigstens 65:1 beträgt.
2. Sichtbarmachungsausrüstung nach Anspruch 1, dadurch gekennzeichnet, daß das Molverhältnis wenigstens 75:1 beträgt.
3. Sichtbarmachungsausrüstung nach Anspruch 1 oder 2, dadurch gekennzeichnet, daß das Zinn(II)-Reduktionsmittel Zinn(II)-Chlorid ist.
4. Sichtbarmachungsausrüstung nach einem der Ansprüche 1 bis 3, dadurch gekennzeichnet, daß sie ferner eine stabilisierende Menge eines Stabilisators aufweist, der unter Gentisinsäure, Gentisylalkohol und deren pharmazeutisch geeigneten Salzen, Ascorbinsäure, Erythorbinsäure, 6-Brom-6-desoxyascorbinsäure, 6-Chlor-6-desoxyascorbinsäure, Reduktinsäure, 5-Methylreduktinsäure und der Nikotinsäure und deren Nikotinamid-Komplexen und deren pharmazeutisch geeigneten Salzen, und deren Gemischen ausgewählt ist.
5. Stirnbarmachungsausrüstung nach einem der Ansprüche 1 bis 4, dadurch gekennzeichnet, daß
(a) der Träger unter Methanhydroxydiphosphonsäure und ihren pharmazeutisch geeigneten Salzen und Gemischen ausgewählt ist und
(b) die Ausrüstung einen Stabilisator enthält, der unter Gentisinsäure, Ascorbinsäure, 6-Brom-6-desoxyascorbinsäure und deren pharmazeutisch geeigneten Salzen und Gemischen ausgewählt ist.
6. Sichtbarmachungsausrüstung nach einem der Ansprüche 1 bis 5, dadurch gekennzeichnet, daß sie
(a) 3 mg Natriumsalz der Methanhydroxydiphosphonsäure,
(b) 0,02 mg Zinn(II)-Chlorid und
(c) 0,25 mg bis 1,0 mg Ascorbinsäure oder deren Natriumsalz aufweist.
7. Sichtbarmachungsausrüstung nach einem der Ansprüche 1 bis 5, dadurch gekennzeichnet, daß sie
(a) 10 mg Natriumsalz der Methandiphosphonsäure,
(b) 0,02 mg Zinn(II)-Chlorid und
(c) 0,25 mg bis 1,0 mg Ascorbinsäure oder deren Natriumsalz aufweist.

**Revendications**

1. Kit de formation d'image, caractérisé en ce qu'il comprend:
(a) un support choisi entre l'acide méthanediphosphonique, l'acide méthanehydroxydiphosphonique et leurs sels pharmaceutiquement acceptables et leurs mélanges; et
(b) une quantité efficace d'un réducteur stanneux, le rapport molaire dudit support à l'étain stanneux étant au moins égal à 65:1.
2. Kit de formation d'image suivant la revendication 1, caractérisé en ce que ledit rapport molaire est d'au moins 75:1.
3. Kit de formation d'image suivant la revendication 1 ou 2, caractérisé en ce que ledit réducteur stanneux est le chlorure stanneux.
4. Kit de formation d'image suivant l'une quelconque des revendications 1 à 3, caractérisé en ce qu'il comprend en outre une quantité à effet stabilisant d'un agent stabilisant choisi entre l'acide gentisique, l'alcool gentisylique et leurs sels pharmaceutiquement acceptables; l'acide ascorbique, l'acide érythorbique, l'acide 6-bromo-6-déoxyascorbique, l'acide 6-chloro-6-déoxyascorbique, l'acide réductique, l'acide 5-méthylréductique, leurs complexes avec l'acide nicotinique, leurs complexes avec le nicotinamide et leurs sels pharmaceutiquement acceptables; et leurs mélanges.
5. Kit de formation d'image suivant l'une quelconque des revendications 1 à 4, caractérisé en ce que
(a) ledit support est choisi entre l'acide méthanehydroxydiphosphonique et ses sels pharmaceutiquement acceptables et leurs mélanges; et
(b) ledit kit comprend un agent stabilisant choisi entre l'acide gentisique, l'acide ascorbique, l'acide 6-bromo-6-déoxyascorbique, leurs sels pharmaceutiquement acceptables et leurs mélanges.
6. Kit de formation d'image suivant l'une quelconque des revendications 1 à 5, caractérisé en ce qu'il comprend:

# 0 096 934

(a) 3 mg des sels de sodium de l'acide méthanehydroxydiphosphonique;
(b) 0,02 mg de chlorure stanneaux; et
(c) 0,25 mg à 1,0 mg d'acide ascorbique ou de son sel de sodium.

7. Kit de formation d'image suivant l'une quelconque des revendications 1 à 5, caractérisé en ce qu'il comprend:

(a) 10 mg des sels de sodium de l'acide méthanediphosphonique;
(b) 0,02 mg de chlorure stanneux; et
(c) 0,25 mg à 1,0 mg d'acide ascorbique ou de son sel de sodium.

9